# FASCICULE DE BREVET EUROPEEN

(11) **EP 2 811 977 B1**
(45) Date de publication et mention de la délivrance du brevet: **01.08.2018**
(21) Numéro de dépôt: 13706652.8
(22) Date de dépôt: 07.02.2013
(51) Int. Cl.: A61K 8/9789, A61Q 19/08

(54) **UTILISATION D'UN EXTRAIT DE FEUILLE DE POMMIER DANS UNE COMPOSITION COSMETIQUE POUR LE RAFFERMISSEMENT DE LA PEAU**
VERWENDUNG EINES APFELBAUMBLATTEXTRAKTES IN EINER KOSMETISCHEN HAUT-STRAFFUNGS ZUSAMMENSETZUNG
USE OF AN APPLE TREE LEAF EXTRACT IN A COSMETIC SKIN-FIRMING COMPOSITION

(30) Priorité: 09.02.2012 FR 1251224
(43) Date de publication de la demande: 17.12.2014
(73) Titulaire: SOCIETE DE RECHERCHE COSMETIQUE SARL, 2314 Luxembourg (LU)
(72) Inventeur: LECONTE, Nadine, F-92600 Asnieres sur Seine (FR); LECLERE, Jacques, F-45500 Saint-Gondon (FR)
(74) Mandataire: Goulard, Sophie
(86) Numéro de dépôt international: PCT/FR2013/050261
(87) Numéro de publication internationale: WO 2013/117866

(56) Documents cités:
- WO-A1-2007/026101
- WO-A2-2006/040766
- FR-A1- 2 916 972
- DATABASE GNPD [Online] MINTEL; juin 2010 (2010-06), "Intensive Renewal Serum", XP002689142, Database accession no. 1345665
- DATABASE GNPD [Online] MINTEL; novembre 2010 (2010-11), "Cellular Renewal Masque", XP002689143, Database accession no. 1442699
- DATABASE GNPD [Online] MINTEL; décembre 2010 (2010-12), "Bio Reform 28 Age Reversal Complex", XP002719906, Database accession no. 1450618
- DATABASE WPI Week 200975 Thomson Scientific, London, GB; AN 2009-R24225 XP002689144, & RU 2 371 929 C1 (KVASENKOV O I) 10 novembre 2009 (2009-11-10)
- Sy Jye Leu ET AL: "Phenolic Constituents of Malus doumeri var. formosana in the Field of Skin care", Biol. Pharm. Bull., vol. 29, no. 4, 1 April 2006 (2006-04-01), pages 740-745, XP055325056,

## Description

La présente invention concerne une nouvelle composition à base d'extrait de feuille de pommier, utilisable en cosmétique, plus particulièrement une composition comprenant un extrait de feuilles de pommier susceptible d'agir efficacement contre les effets du vieillissement cutané.

La peau comprend des couches superficielles, à savoir l'épiderme, et des couches plus profondes, le derme et l'hypoderme, et chacune possède des propriétés spécifiques permettant à l'ensemble de réagir et s'adapter aux conditions de son environnement. L'épiderme, qui est composé de trois types de cellules, à savoir des kératinocytes (90% des cellules épidermiques), des mélanocytes (2 à 3% des cellules épidermiques) et des cellules de Langerhans, constitue la couche externe et joue un rôle fondamental pour assurer la protection et le maintien d'une bonne trophicité. Le derme sert de support à l'épiderme et est principalement constitué de fibroblastes et d'une matrice extracellulaire essentiellement à base de collagène et d'élastine. Les fibres de collagène contribuent à la texture et la tonicité de la peau et l'élastine est responsable de son élasticité. D'autres cellules, comme les macrophages et les leucocytes, sont également présentes dans la couche du derme. L'hypoderme, qui est la couche la plus profonde de la peau, contient les adipocytes qui produisent des lipides pour que le tissu sous-cutané fabrique une couche grasse protégeant les muscles, les os et les organes internes contre les chocs.

Le vieillissement de la peau est un processus qui peut être intrinsèque et naturel, ou extrinsèque c'est-à-dire provoqué par l'environnement, y compris l'exposition au soleil, les variations de températures et les radicaux libres. Le vieillissement de la peau provoque notamment une diminution des synthèses protéiques (collagène, élastine), une diminution de la synthèse des protéo-glycanes, ainsi qu'une accumulation des métalloprotéinases de type MMP3. Les premiers signes du vieillissement de la peau, tels que les rides et ridules, peuvent s'accompagner de marques pigmentaires, d'une diminution de l'épaisseur de la peau. Ce vieillissement de la peau se traduit notamment par une perte de fermeté, une modification de l'ovale du visage, une moindre élasticité, une diminution de l'hydratation.

On connaît diverses substances d'origine végétale que l'on peut incorporer dans des compositions destinées au traitement des symptômes du vieillissement de la peau. Ainsi, le brevet FR 2.761.607 décrit une composition comportant un dérivé de silanol méthylé et un dérivé d'une protéine végétale hydrolysée, additionnée le cas échéant d'un dérivé de vitamine C. La demande WO 2004062637 décrit une composition cosmétique et/ou dermatologique à base d'inhibiteurs de métallo-protéinases matricielles, en particulier d'extrait de Siegesbeckia, et de lipopeptides permettant le traitement et la prévention des signes du vieillissement cutané tels que l'apparition de rides et la perte d'élasticité de la peau. Une composition à base d'extrait de graines de mimosa, permettant d'agir contre l'apparition des rides et la perte d'élasticité de la peau, est décrite dans la demande WO 2007144518.

On a aussi proposé d'utiliser des extraits d'arbres fruitiers, par exemple l'écorce, les feuilles et les fruits, en fonction des effets recherchés, notamment de diverses plantes de la famille des Rosaceae, telles que poiriers et pommiers.

Le pommier (Pyrus malus) est un des arbres fruitiers les plus cultivés dans le monde, en Chine, en Amérique du Nord et en Europe, dans des régions généralement tempérées, produisant de nombreuses variétés de fruits.

Le fruit du pommier a été utilisé depuis de nombreuses années en cosmétique, par exemple pour préparer des masques que l'on peut appliquer momentanément sur la peau, généralement en vue d'un effet d'hydratation de la peau, et aussi d'un effet légèrement astringent destiné à resserrer les pores de la peau. Diverses compositions cosmétiques contenant des extraits de pomme ont été proposées en cosmétique, et par exemple des extraits de fruit de Pyrus malus sont utilisés dans des shampooings et dans des crèmes de soins cosmétiques. La pomme contient aussi des pectines qui peuvent être utiles en raison de leur effet hypocholestérolémiant.

Ainsi, le brevet FR 2.719.473 décrit une composition cosmétique comprenant un extrait sec de jus de fruit de Rosaceae tel que poire, pomme, prune, abricot, etc, riche en acide malique, en acide citrique et en sorbitol présentant un effet antiradicalaire et un effet favorisant le renouvellement cellulaire. La pomme contient aussi des pectines qui peuvent être utiles en raison de leur effet hypocholestérolémiant, ainsi des polyphénols à action anti-oxydante. La demande WO 01 /24806 décrit des extraits secs de branches de pommier susceptibles de procurer des effets amincissants. Le brevet EP 1.338.270 décrit l'utilisation de fractions phénoliques d'extraits de fruits de la famille des Rosaceae, notamment la pomme et plus particulièrement l'espèce Malus sylvestris, dont la fraction polyphénolique est riche en dihydrochalcones permettant la préparation de compositions utiles en cosmétique et en nutraceutique pour limiter la surcharge pondérale en raison de l'effet de diminution de l'absorption des sucres par les tissus cutanés résultant principalement de la présence de phloridzine dans les extraits. Si quelques compositions mentionnent un extrait de feuille de pommier parmi les divers ingrédients, comme le « Intensive Renewal Serum » décrit dans la base GNPD (n° 1345665 - juin 2010), il s'agit d'agent de conditionnement sans effet spécifique. Peu d'études ont été consacrées à l'utilisation des feuilles de pommier en cosmétique.

Les études réalisées par la demanderesse sur des fibroblastes humains en culture ont montré de manière inattendue qu'un extrait de feuilles de pommier tel que décrit dans les revendications, présente un effet inhibiteur significatif des élastases permettant de l'utiliser efficacement contre le vieillissement cutané. On sait que les diverses parties d'une même plante peuvent contenir des substances possédant des activités différentes et les études ont montré que l'effet anti-élastase des extraits de feuilles ne se retrouve ni dans les extraits de fleurs ni dans les extraits de fruits.

La présente invention a donc pour objet l'utilisation d'un extrait de feuilles de pommier tel que décrit dans les revendications pour la préparation d' une composition cosmétique topique utile pour le raffermissement de la peau, en une concentration appropriée, améliorant la résistance de la peau aux effets du vieillissement par un effet inhibiteur de l'élastase.

L'invention a encore pour objet un procédé de traitement cosmétique non thérapeutique de la peau par application d'une composition à base d'extrait de feuilles de pommier tel que décrit, sur la zone de la peau nécessitant un tel traitement, notamment la face et le cou.

Plus particulièrement, il a été démontré qu'un extrait de feuilles de pommier selon l'invention
- présente une parfaite innocuité vis-à-vis des fibroblastes humains en culture ;
- inhibe significativement l'activité de l'élastase au niveau des fibroblastes humains en culture ;
- augmente significativement les glycosaminoglycannes ;
- augmente significativement la synthèse de l'élastine ;
- augmente significativement la néosynthèse des collagènes totaux ;
- présente un effet antiradicalaire marqué.

L'augmentation des glycosaminoglycannes contribue à une meilleure fixation de l'eau liée dans la couche épidermique, favorisant une meilleure fermeté de la peau et lui donnant un aspect repulpé. L'inhibition de l'élastase simultanément avec l'augmentation de la synthèse de l'élastine, contribue à une meilleure élasticité et une plus grande fermeté de la peau.

Les extraits de feuilles de pommier suivant l'invention peuvent être obtenus à partir de feuilles de pommiers de la famille des Rosaseae, de préférence des espèces Pyrus malus ou Malus sylvestris Mill., Malus domestica, Malus pumila, Malus floribunda, Malus coronaria, etc, dont on connaît un grand nombre de variétés.

Les extraits utilisés dans la présente invention sont obtenus à partir des feuilles de pommier, de préférence par séchage et broyage des feuilles, suivi d'une macération ou d'une extraction par un solvant et séparation des phases liquides et solides. Le solvant d'extraction éventuellement utilisé est de préférence l'eau ou un mélange eau/alcool. La méthode d'extraction préférée est la macération à une température de 25 à 40°C. Suivant une variante on peut procéder par percolation au moyen d'une poudre de granulométrie appropriée.

Les feuilles sont de préférence récoltées vers la période de l'année correspondant au début de l'automne, en fin de fructification, là où elles sont le plus concentrées en actifs.

Suivant une forme avantageuse de réalisation, l'extrait utilisable dans la composition de l'invention est obtenu à partir des feuilles séchées et réduites en poudre par broyage, et macération dans de l'eau pendant une durée de 1 à 3 jours, à une température comprise entre 25 et 30°C. Le produit est ensuite décanté et filtré et la masse solide est exprimée. Les liqueurs sont jointes et le liquide est stabilisé au moyen d'un conservateur approprié, par exemple l'alcool benzylique et l'acide déhydroacétique.

On peut éventuellement préparer un extrait sec à partir de l'extrait aqueux, auquel on peut ajouter avantageusement de la maltodextrine pour éviter le mottage.

Les extraits de feuilles de pommier utilisés dans les compositions suivant la présente invention sont sous forme d'extraits aqueux, de préférence à partir des feuilles séchées et broyées à température ambiante.

L'extrait aqueux de feuilles de pommier utilisé dans la présente invention et utilisé dans les exemples ci-après, a été obtenu à partir de feuilles de pommiers biologiques de Provence (Pyrus malus) et se présente sous la forme d'un liquide de couleur orange à ambré, d'odeur caractéristique, soluble à 10% dans l'eau et dans l'éthanol, présentant les caractéristiques physico-chimiques indiquées ci-après :
- pH 4,5 - 6,5
- indice de réfraction à 22°C 1,330 - 1,380
- densité à 20°C 0,950 - 1,050
- matières sèches résiduelles 1,5 - 3,0 %

L'extrait de la présente invention est relativement riche en polysaccharides, en flavonoïdes (il contient notamment de la rutine, mais les analyses n'ont pas fait apparaître de phloridzine, sinon à l'état de traces) et en sucres. En tout état de cause il contient, comme le montrent les résultats des analyses ci-après :
- flavonoïdes (exprimés en rutine) 0,5 - 4,0 g/l
- polysaccharides (matières sèches) 2,0 - 8,0 %
- sucres totaux (matières sèches) 25,0 - 40,0 %

Dans la composition selon l'invention, la teneur en extrait de feuilles de pommier peut être comprise entre 0,5 et 20%, et de préférence entre 1 et 10% en poids par rapport au poids total de la composition, pour procurer les meilleurs effets anti-vieillissement.

Les extraits de feuilles de pommier peuvent être utilisés avantageusement sous une forme encapsulée dans des liposomes.

Suivant une technique connue dans la fabrication des compositions cosmétiques, les liposomes sont constitués par des petites sphères creuses, de diamètre généralement inférieur à 500 nm, dont la paroi est formée d'une double couche de lipides tels que des glucolipides ou des phospholipides. Ils peuvent être obtenus par exemple par traitement aux ultrasons d'un mélange d'un soluté aqueux et de lipides. Les lipides (phospholipides ou glucolipides) se réorganisent dans une configuration où l'énergie de l'ensemble est minimale, donc thermo-dynamiquement la plus stable. Les liposomes sont utilisés dans l'industrie cosmétique pour délivrer des composés à l'intérieur des cellules lorsque le vésicule fusionne avec la membrane plasmique.

Suivant une forme de réalisation de l'invention, une partie au moins des extraits est encapsulée dans des vésicules du type liposome.

Les compositions conformes à la présente invention sont de préférence administrables par voie topique. Suivant la terminologie classique, l'administration par voie topique désigne toute méthode consistant à appliquer la substance ou la composition directement sur la peau, sur la zone nécessitant le traitement.

Conformément à la présente invention, la composition administrable par voie topique peut avantageusement contenir, outre les composants de base décrits ci-dessus, une ou plusieurs autres substances connues pour exercer des effets complémentaires bénéfiques pour la peau, et plus particulièrement le tocophérol, la vitamine A (rétinol), l'acide rétinoïque, des agents bactéricides, du mono-méthylsilanol, de la proline, ou encore du beurre de Karité.

On peut aussi ajouter à la composition des extraits ou composés connus pour faciliter la pénétration des principes actifs de la composition à travers l'épiderme, par exemple des saponosides du type hédéragénine.

Les compositions cosmétiques et pharmaceutiques conformes à la présente invention, sont destinées de préférence à une administration topique, et contiennent donc des supports et excipients couramment utilisés dans des compositions de ce type telles que des émulsions H/E ou E/H, des crèmes, des gels ou des lotions. Dans le cas des émulsions, la phase grasse peut représenter entre 10 et 60% environ du poids de la composition, la phase aqueuse entre 10 et 80% environ et l'agent émulsionnant entre 2 et 20%, le reste étant constitué par les composants de base indiqués ci-dessus et les autres composants mentionnés ci-après.

La composition peut encore contenir diverses substances et excipients choisis en fonction de leurs propriétés connues et de la forme galénique envisagée. Ainsi, on peut incorporer dans la composition des conservateurs, des agents émulsionnants, des agents viscosants, des épaississants, des gélifiants, des agents de chélation, des antioxydants, des agents hydratants, des tensioactifs, des parfums, des huiles, des lipides, un solvant spécifique ainsi que de l'eau et divers additifs destinés à améliorer les propriétés physiques de la composition.

On peut choisir l'agent émulsionnant parmi des polymères carboxyvinyliques à haut poids moléculaire, des polysorbates, des esters de sorbitan et en particulier un monostéarate tel que le Span 60®, un tristéarate, un monopalmitate, et un laurate de sorbitan. On peut encore utiliser d'autres agents émulsionnants tels que divers dérivés d'acide stéarique ou palmitique, et par exemple le stéarate de PEG 100®, des mono- ou diglycérides d'acide stéarique ou palmitique, un ester de polyglycérol, un ester gras d'acide citrique ou tartrique, un stéarate de propylène glycol auto-émulsionnable, ou encore le polyglycéryl-2-sesquioléate, l'éther cétylique de polyoxyéthylène, un polyglucoside de siloxane, ou une silicone émulsionnable. On peut aussi utiliser un émulsionnant choisi parmi les lécithines hydrogénées, les stérols végétaux (par exemple de soja), des lipoprotéines et des sphingolipides.

Les agents viscosants utilisés dans les compositions de l'invention peuvent être choisis parmi divers polymères d'acide acrylique, un copolymère d'acrylate et de laurate d'acryloyle, une gomme cellulose, une gomme arabique, le Sclerotium gum, le pullulan, un mucilage de Gleditsia, une silice, des polymères carboxyvinyliques, un silicate d'aluminium et de magnésium, et on peut utiliser par exemple la silice colloïdale vendue sous la marque Aerosil 200® ou un acide polyacrylique réticulé tel que le Carbopol 940®.

Les gélifiants ou épaississants peuvent être choisis par exemple parmi les polyacrylamides, des acrylates comme le Pemulen®, les dérivés de cellulose comme l'hydroxypropyl cellulose, ou les gommes naturelles telles qu'une gomme Xanthane.

Les agents chélatants permettent d'améliorer la stabilité des produits dans le temps et peuvent être choisis par exemple parmi l'EDTA disodique ou tétra-sodique, l'acide oxalique ou l'acide galactarique, des dérivés de l'acide panthétique ou de l'acide étidronique, ainsi que l'acide phytique.

Les agents hydratants utilisés peuvent être choisis par exemple parmi un polyol, le sorbitol, le maltitol, le xylitol, le pentaérythritol, le butylène glycol, l'alcool D-panthotényl (Panthenol), les polyacrylates et polyméthacrylates de glycéryle, le glycérol ou des dérivés de glycérol, ou encore une argile hydratante comme le Veegum HV (silicate de magnésium / aluminium) qui a aussi pour effet de stabiliser l'émulsion. On peut encore ajouter des émollients tels qu'un malate d'alkyle, l'isohexadécane, des triglycérides d'acide caprique ou caprylique, l'alcool béhénylique, etc.

Les conservateurs usuels de la technique des compositions dermatologiques ou cosmétologiques peuvent être utilisés dans l'invention, et par exemple l'acide déhydroacétique et son sel de sodium, l'acide benzoïque et un p-hydroxybenzoate d'alkyle tel que le p-hydroxy-benzoate de méthyle (Méthylparaben), un alcool tel que le phénoxy-éthanol, l'alcool benzylique ou encore la chlorphénésine ou l'imidazolidinyl urée, ainsi que des dérivés d'acide vanillique comme l'acide anisique, l'acide lévulinique, l'acide gluconique et le gluconate de calcium.

Les constituants de la phase grasse, c'est-à-dire les huiles et lipides, peuvent être choisis parmi l'huile de jojoba, l'huile de maïs, l'huile de noisettes, l'huile d'amandes douces, l'huile de coco hydrogénée, l'huile de carthame, des glycérides d'acides gras saturés, un triglycéride d'acides caprique et caprylique, l'acide stéarique, l'acide palmitique, le stéarate d'octyle, le palmitate de glycéryle, le palmitate d'octyle, le 2-octyl-dodécanol, le polyéthylène glycol, l'adipate d'éthyl-2 hexyle, ou encore des huiles de silicones telles que le méthyl phényl polysiloxane, la diméthicone, la cyclométhicone et la phényl diméthicone.

La composition peut aussi contenir un solvant choisi en fonction des composants utilisés et de la forme d'administration envisagée. Le solvant peut être par exemple de l'eau, et de préférence de l'eau déminéralisée, ou un solvant spécifique tel que le propylène glycol, le butylène glycol-1,3, le butylène glycol-1,4, un éther de diéthylène glycol, ou un alcool tel que l'éthanol.

Des agents de protection contre les rayons ultraviolets peuvent aussi être avantageusement incorporés dans les compositions, et par exemple des filtres solaires UV-A et UV-B hydrophiles ou lipophiles. On peut aussi utiliser des pigments formant écran anti-ultraviolet, comme par exemple le dioxyde de titane, l'oxyde de zinc et l'oxyde de zirconium.

Le pH de la composition est de préférence compris entre 5,3 et 7,5, et peut être ajusté, selon les compositions, par addition d'un acide tel que l'acide citrique ou d'une base telle que l'hydroxyde de sodium ou de potassium.

La composition conforme à la présente invention peut être présentée sous les formes classiquement utilisées pour une application topique, c'est-à-dire sous forme de gel, lotion, émulsion (en particulier crème ou lait), masque ou pommade, contenant des excipients et supports usuels compatibles et pharmaceutiquement acceptables. Ces formes d'administration par voie topique sont préparées par les techniques connues, et par exemple, dans le cas d'une crème, par dispersion d'une phase grasse dans une phase aqueuse pour obtenir une émulsion huile dans eau, ou inversement pour préparer une émulsion eau dans huile. Dans le cas de crèmes, on préfère utiliser des émulsions à structure lamellaire contenant peu de produits éthoxylés ou n'en contenant pas du tout. L'extrait de pomme utilisé dans l'invention est introduit dans la phase aqueuse ou alcoolique.

A titre d'exemple, on peut préparer des compositions topiques conformes à l'invention sous forme de crèmes, de lotions ou de gels, utilisables en une ou plusieurs applications quotidiennes, la durée du traitement pouvant être généralement de l'ordre de un à trois mois ou davantage, les compositions pouvant être utilisées sans inconvénient de manière continuelle.

Les exemples de compositions donnés ci-après illustrent l'invention. Sauf indication contraire, les parties et pourcentages sont indiqués en poids.

### Exemple 1

Une crème raffermissante pour les soins du visage, à base d'extrait de feuilles de pommier ayant la composition indiquée ci-après est préparée suivant les techniques usuelles :

**Phase A**

| | | |
|---|---|---|
| Eau déminéralisée | q.s.p. | 100,00 |
| Chlorphénésine | | 0,25 |
| EDTA disodique | | 0,10 |
| Butylène glycol-1,3 | | 4,00 |
| Glycérine | | 3,00 |
| Gomme xanthane | | 0,30 |
| Veegum HV | | 0,20 |

**Phase B**

| | |
|---|---|
| Tocophérol | 0,70 |
| PEG 100 stearate (and) glycerol stearate | 4,50 |
| Alcool béhénylique | 2,50 |
| Triglycérides caprique / caprylique | 2,00 |
| Propyl heptyl caprylate | 1,50 |
| Beurre de Karité | 0,75 |
| Stearyl isononanoate | 1,00 |
| Glycéryl undecylate | 0,50 |

**Phase C**

| | |
|---|---|
| Silicate de magnésium | 1,00 |

**Phase D**

| | |
|---|---|
| Hyaluronate de sodium | 0,10 |
| Eau déminéralisée | 5,00 |

**Phase E**

| | |
|---|---|
| Extrait aqueux de feuilles de pommier | 2,50 |
| Extrait d'Echinacée | 2,00 |

**Phase F**

| | |
|---|---|
| Parfum | 0,30 |

La phase aqueuse A est chauffée à 80°C en formant un gel tandis que la phase grasse B est chauffée à environ 80°C pour faire fondre les composants, puis les deux phases sont mélangées soigneusement sous agitation. La phase C est ajoutée au mélange à la température d'environ 65°C, puis les phases D et E à 50°C environ. Le mélange est ramené à environ 40°C et le parfum est ajouté.

Cette crème peut être utilisée par application sur les zones de la peau du visage et du cou à traiter, une à deux fois par jour, pendant une période de un à trois mois.

### Exemple 2

Un lait pour le corps, à base d'extrait de feuilles de pommier biologique de Provence ayant la composition indiquée ci-après est préparé suivant les techniques usuelles :

**Phase A**

| | | |
|---|---|---|
| Eau déminéralisée | q.s.p. | 100,00 |
| Acide galactarique | | 0,10 |
| Dehydroacétate de sodium | | 0,15 |
| Alcool benzylique | | 0,20 |
| Butylène glycol | | 5,00 |
| Gomme xanthane | | 0,30 |

**Phase B**

| | |
|---|---|
| Arachidyl glucoside | 3,00 |
| Glycéryl stéarate citrate | 1,00 |
| Alcool béhénylique | 2,00 |
| Triglycérides caprique / caprylique | 4,00 |
| Huile de noisette | 2,50 |
| Glyceryl undecylate | 0,35 |

**Phase C**

| | |
|---|---|
| Silicate de magnésium | 1,00 |

**Phase D**

| | |
|---|---|
| Extrait aqueux de feuilles de pommier | 3,00 |
| Extrait de graines d'Amaranthe | 1,50 |

**Phase E**

| | |
|---|---|
| Hyaluronate de sodium | 0,10 |
| Parfum | 0,50 |

La phase aqueuse A est chauffée à 80°C en formant un gel tandis que la phase grasse B est chauffée à environ 80°C, puis les deux phases sont mélangées soigneusement sous agitation. La phase C est ajoutée au mélange à la température d'environ 60°C, puis la phase D à 50°C environ. Enfin le parfum est ajouté à environ 40°C.

Ce lait peut être utilisé par application sur les zones de la peau à traiter, une à deux fois par jour, pendant une période de un à trois mois.

### Exemple 3

Un gel raffermissant ayant la composition ci-dessous est préparé par les techniques usuelles.

**Phase A**

| | | |
|---|---|---|
| Eau déminéralisée | q.s.p. | 100,00 |
| Acide phytique | | 0,10 |
| Dehydroacétate de sodium | | 0,15 |
| Alcool benzylique | | 0,30 |
| Acide lévulinique | | 0,15 |
| Butylène glycol | | 5,00 |
| Gomme xanthane | | 0,40 |

**Phase B**

| | |
|---|---|
| Caféine | 2,00 |
| Gel de Gleditsia (poudre) | 1,00 |
| Eau | 10,00 |

**Phase C**

| | |
|---|---|
| Extrait de lierre grimpant | 2,00 |
| Extrait de Coleus | 1,00 |
| Extrait de feuilles de pommier | 5,00 |
| Parfum | 0,50 |

Les phases A et B, puis C, sont chauffées et mélangées pour former un gel.

Ce gel peut être utilisé par application sur les zones de la peau à traiter, une à deux fois par jour, pendant une période de un à trois mois.

### Exemple 4

### Evaluation de la cytotoxicité

La cytotoxicité des extraits de feuilles de pommier suivant l'invention a été étudiée vis-à-vis de fibroblastes humains en culture.

Des cultures de fibroblastes ont été établies à partir de prélèvements de peau humaine et ont été amplifiées en milieu de culture cellulaire RPMI 1640 supplémenté en sérum de veau foetal, L-glutamine et pénicilline/streptomycine. Les essais sont effectués sur des fibroblastes entre le 4^{ème} et le 6^{ème} passage. Les fibroblastes sont ensemencés sur des plaques 6 puits à raison de 10⁵ cellules par ml. Ils sont ensuite incubés pendant 24 heures.

Quatre lots ont été constitués :
Lot n° 1 : témoin ne recevant aucun produit.
Lot n° 2 : traité avec l'extrait aqueux de feuilles de pommier à 0,1 %
Lot n° 3 : traité avec l'extrait aqueux de feuilles de pommier à 0,5 %
Lot n° 4 : traité avec l'extrait aqueux de feuilles de pommier à 1,0 %

Chaque lot a été maintenu au contact des fibroblastes humains en culture pendant 24 heures.

La viabilité cellulaire est déterminée par le test de réduction au bleu de Formazan (test MTT) après 24 heures d'incubation des cellules de fibroblastes.

Après incubation des cellules avec chacun des lots de produit à l'étude, les puits contenant les cellules sont vidés par retournement doux et le tapis cellulaire est rincé avec le milieu de culture. On distribue dans tous les puits 200 µl d'une solution de MTT (bromure de 3-(4,5-diméthylthiazol-2-yl)-2,5-diphényltétrazolium) diluée au moment de l'emploi (5 mg/ml), puis les plaques sont incubées à 37°C pendant 3 heures.

Les puits sont à nouveau vidés par retournement doux et remplacés pendant 1 minutes par 200 µl d'une solution de formo-calcium pour fixer le tapis cellulaire. On observe alors la formation de cristaux de bleu de Formazan. Les cellules sont ensuite lysées et les cristaux de bleu de Formazan sont dissous par addition de 200 µl de diméthylsulfoxyde (DMSO). Après homogénéisation de la coloration par agitation, la densité optique (DO) des plaques est lue, au moyen d'un spectrophotomètre à 570 nm, ce qui permet de connaître la quantité relative de cellules vivantes et actives métaboliquement.

Les valeurs de la densité optique (DO) après 24 heures de contact sont regroupées dans le tableau ci-dessous.

| Substance | Densité optique DO 570 nm | % |
|---|---|---|
| Lot n°1 (témoin) | 0,525 ± 0,04 | - |
| Lot n°2 (0,1 %) | 0,530 ± 0,03 | ns |
| Lot n°3 (0,5 %) | 0,540 ± 0,06 | ns |
| Lot n°4 (1,0 %) | 0,537 ± 0,05 | ns |

Ces résultats montrent que l'extrait de feuilles de pommier utilisé dans l'invention ne présente aucune cytotoxicité vis-à-vis des fibroblastes humains en culture aux concentrations étudiées.

### Exemple 5

L'évaluation de l'activité anti-élastase de l'extrait de feuilles de pommier utilisé dans l'invention, au niveau des fibroblastes humains en culture, a été effectuée comme indiqué ci-après.

L'évaluation a été faite sur les mêmes fibroblastes humains en culture que ceux de l'Exemple 4 ci-dessus.

Les lots témoin et extrait à la concentration de 1,0% sont constitués comme suit :
Lot n° 1 : témoin ne recevant aucun produit.
Lot n° 2 : traité avec l'extrait aqueux de feuilles de pommier à 1 % + SANA.

L'essai est réalisé en triplicate après 24 heures de contact des cellules de fibroblastes avec chaque lot en présence de substrat N-succinyl trialanine paranitroanilide (SANA).

Les essais sont réalisés sur des fibroblastes entre le 4^{ème} et le 6^{ème} passage. Les fibroblastes sont ensemencés sur des plaques multipuits à raison de 10⁵ cellules / puits. Ils sont ensuite incubés avec 2% de SVF et RMPI pendant 24 heures. Le SVF est ensuite remplacé par 0,2% de BSA et les cellules sont incubées pendant 24 heures en présence des produits à l'étude. En fin de traitement, les cellules sont récupérées par grattage et les enzymes sont extraites du culot cellulaire en utilisant 0,1% de Triton X-100 dans un tampon Tric-HCl à pH 8, Brij 35 0,1% et 20 µl d'une solution de N-succinyl trialanine paranitroanilide (SANA, 125 mM) dans de la N-éthylpyrrolidone. La réaction est initiée à 37°C et stoppée par ajout de 50 µl d'acide acétique. La densité optique est mesurée à 410 nm au moyen d'un spectrophotomètre.

Les unités enzymatiques sont définies en nM de nitroaniline libérée par heure et par 10⁵ cellules.

Les résultats sont regroupés dans le tableau ci-dessous.

| Substance | Activité de l'élastase (nM de SANA hydrolysé / heure / 10⁵ cellules) | % |
|---|---|---|
| Lot n°1 (témoin) | 61,7 ± 4,2 | - |
| Lot n°2 (1,0 %) | 50,6 ± 2,6 | - 18 |

Ces résultats montrent que l'extrait de feuilles de pommier utilisé dans l'invention, à la concentration de 1,0%, inhibe significativement, de -18%, l'activité de l'élastase au niveau des fibroblastes humains en culture,.

### Exemple 6

L'évaluation de l'effet de l'extrait de feuilles de pommier utilisé dans l'invention sur la synthèse des glycosaminoglycannes, a été effectuée comme indiqué ci-après.

Les lots (témoin et extraits à 0,1%, 0,5% et 1,0%) et les cultures de fibroblastes humains sont les mêmes que dans l'Exemple 4 mais le milieu de culture RPMI 1640 est supplémenté en sérum de veau foetal, L-glutamine et genta-mycine.

Les fibroblastes sont ensemencés dans des boîtes multipuits (6 puits) à raison de 2x10⁵ cellules par ml dans 3 ml de milieu de culture, et sont ensuite maintenus 24 heures en étuve sous CO₂.

La méthode consiste à incorporer un précurseur radioactif, la [³H]-glucosamine, ajouté aux cultures 18 heures avant la récupération des cellules. Le temps de contact des cellules avec les produits à tester est de 24 heures à 37°C.

Après élimination du milieu par aspiration, les cellules sont lavées avec du milieu sans sérum, puis détachées du support par grattage, centrifugées (600 g, 5 minutes).

Le culot obtenu permet de réaliser la néosynthèse des glycosaminoglycannes (GAGs) et le dosage des protéines totales.

Une fraction du culot est traitée par la pronase (1 mg/ml, 24 heures, 60°C), puis la réaction est stoppée par refroidissement des tubes. Les protéines sont précipitées par du TCA (12%, 4°C) pendant la nuit. Les précipités sont ensuite centrifugés (12.000 g, 30 minutes) et les surnageants contenant les GAGs sont récupérés, dialysés contre de l'eau ultra pure (MilliQ Plus), puis lyophilisés. Les lyophilisats sont mis en suspension dans un tampon Tris-HCl pH 7,4 contenant des inhibiteurs de protéase. Un aliquote de 50 µl est prélevé pour le comptage de la radioactivité incorporée dans les GAGs totaux.

Les résultats sont regroupés dans le tableau ci-dessous.

| Substance | [³H]-glucosamine (cpm) | % |
|---|---|---|
| Lot n°1 (témoin) | 526 ± 42 | - |
| Lot n°2 (0,1 %) | 603 ± 33 | + 15 |
| Lot n°3 (0,5 %) | 618 ± 59 | + 17 |
| Lot n°4 (1,0 %) | 631 ± 60 | + 20 |

Ces résultats montrent que l'extrait de feuilles de pommier, aux concentrations testées, stimule significativement la néosynthèse des glycosaminoglycannes totaux au niveau des fibroblastes humains en culture.

### Exemple 7

L'évaluation de l'effet de l'extrait de feuilles de pommier utilisé dans l'invention sur la synthèse de l'élastine, a été effectuée comme indiqué ci-après.

On utilise les mêmes lots et les mêmes cellules de fibroblastes que dans l'Exemple 6.

Le dosage de l'élastine est réalisé au moyen d'un kit de dosage Fastin® (Biodye Science).

Les résultats sont regroupés dans le tableau ci-dessous, l'élastine étant mesurée en µg/mg de protéine.

| Substance | Elastine (µg/mg) | % |
|---|---|---|
| Lot n°1 (témoin) | 13,3 ± 0,9 | - |
| Lot n°2 (0,1 %) | 15,4 ± 1,0 | + 16 |
| Lot n°3 (0,5 %) | 15,8 ± 1,7 | + 19 |
| Lot n°4 (1,0 %) | 16,4 ± 1,2 | + 23 |

Ces résultats montrent que l'extrait de feuilles de pommier, aux concentrations testées, stimule significativement la synthèse de l'élastine au niveau des fibroblastes humains en culture.

### Exemple 8

L'extrait de feuilles de pommier utilisé dans l'invention exerce un effet sur la néosynthèse du collagène comme indiqué ci-après.

L'essai a été effectué avec les mêmes lots et les mêmes fibroblastes en culture que dans l'Exemple 7.

Après incubation, les fibroblastes sont récupérés par centrifugation, puis les culots obtenus sont digérés par les collagénases dans l'acide acétique pendant 24 heures à 4°C. Après centrifugation, les collagènes sont précipités par le chlorure de sodium. L'hydroxyproline est dosée par mesure de la fluorescence après séparation par HPLC en phase inverse suivant une technique connue.

Les résultats sont regroupés dans le tableau ci-dessous.

| Substance | Hydroxyproline (mg/L) | % |
|---|---|---|
| Lot n°1 (témoin) | 2,96 ± 0,19 | - |
| Lot n°2 (0,1 %) | 3,45 ± 0,14 | + 17 |
| Lot n°3 (0,5 %) | 3,52 ± 0,11 | + 19 |
| Lot n°4 (1,0 %) | 3,67 ± 0,24 | + 24 |

Ces résultats montrent que l'extrait de feuilles de pommier, aux concentrations testées, stimule significativement la néosynthèse des collagènes totaux au niveau des fibroblastes humains en culture, caractéristique d'un effet de régénération cutanée.

### Exemple 9

L'activité antiradicalaire a été mise en évidence par une étude sur des épidermes humains reconstitués (Skinethics®) décrits par exemple par M. Rosdy, "Reconstituted epidermis for testing", Cosmetics and Toiletries Manuf. Worldwide, Aston Publ. Group. 223-226 (1994).

Suivant cette technique, des épidermes sont formés à partir de kératinocytes d'origine humaine ensemencés sur des filtres en polycarbonate dans un milieu défini (MCDB 153 modifié) et supplémenté, les cellules étant cultivées pendant 14 jours à l'interface air/liquide.

L'activité antiradicalaire a été évaluée par dosage du malondialdéhyde (MDA) après son induction par l'hypoxanthine / xanthine oxydase. Le dosage du MDA, qui est l'un des marqueurs essentiels de la cytotoxicité par les processus oxydatifs et le stress, permet de mesurer l'activité antiradicalaire.

Cinq lots ont été constitués :
Lot n° 1 : épiderme témoin ne recevant aucun produit.
Lot n° 2 : témoin positif traité par hypoxanthine / xanthine oxydase.
Lot n° 3: épiderme traité par Vitamine E + hypoxanthine / xanthine oxydase.
Lot n° 4 : épiderme traité avec l'extrait aqueux de feuilles de pommier à 1,0 %
Lot n° 5 : épiderme traité avec l'extrait aqueux de feuilles de pommier à 1,0 % + hypoxanthine / xanthine oxydase.

Après 24 heures de traitement, les homogénats cellulaires sont remis en suspension dans un tampon approprié à pH 8, on laisse incuber 1 heure dans l'obscurité, on ajoute du n-butanol et on centrifuge pour récupérer la phase supérieure afin de doser le MDA par fluorescence après séparation du complexe MDA-TPA par HPLC.

Le dosage des protéines est fait suivant la méthode de Bradford, par spectrophotométrie.

On détermine ainsi, par dosage du MDA, la lipoperoxydation physiologique, et la lipoperoxydation provoquée par l'hypoxanthine / xanthine oxydase. Les résultats sont regroupés dans les tableaux ci-après.

**Lipoperoxydation physiologique :**

| Substance | MDA (µM/mg protéine) | Diminution du MDA % |
|---|---|---|
| Lot n°1 (témoin) | 713 ± 57 | - |
| Lot n°4 (1,0 %) | 592 ± 62 | - 17 |

Ces résultats montrent que l'extrait de l'invention procure une protection significative vis-à-vis de la lipoperoxydation physiologique puisque la diminution du taux de MDA est de 17%.

**Lipoperoxydation provoquée :**

| Substance | MDA (µM/mg protéine) | Diminution du MDA % |
|---|---|---|
| Lot n°1 (témoin) | 713 ± 57 | - |
| Lot n°2 (témoin positif) | 1001 ± 85 | + 40 |
| Lot n°3 | 650 ± 48 | - 35 |
| Lot n°5 | 780 ± 39 | - 22 |

Ces résultats montrent une protection significative de l'extrait de l'invention vis-à-vis de la lipoperoxydation provoquée par l'hypoxanthine / xanthine oxydase.

## Revendications

1. Utilisation d'un extrait aqueux de feuille de pommier comprenant des flavonoïdes, des polysaccharides et des sucres à raison de :
- flavonoïdes (exprimés en rutine) 0,5 -4,0 g/l
- polysaccharides (matières sèches) 2,0-8,0 %
- sucres totaux (matières sèches) 25,0 -40,0 %
pour la préparation d'une composition cosmétique pour le raffermissement de la peau.

2. Utilisation selon la revendication 1, **caractérisée en ce que** ledit extrait est obtenu à partir de feuilles de pommiers des espèces Pyrus malus ou Malus sylvestris Mill., Malus domestica, Malus pumila, Malus floribunda, ou Malus coronaria.

3. Utilisation selon l'une quelconque des revendications précédentes, **caractérisée** en ce ledit extrait a les caractéristiques suivantes :
- pH 4,5-6,5
- indice de réfraction à 22°C 1,330 - 1,380
- densité à 20° C 0,950 - 1,050
- matières sèches résiduelles 1,5 -3,0 %

4. Utilisation selon l'une quelconque des revendications précédentes, **caractérisée en ce que** la teneur en extrait de feuilles de pommier est comprise entre 0,5 et 20%en poids par rapport au poids total de la composition.

5. Utilisation selon la revendication 4, **caractérisée en ce que** la teneur en extrait de feuilles de pommier est comprise entre 1 et 10%en poids par rapport au poids total de la composition.

6. Procédé de traitement cosmétique non thérapeutique pour le raffermissement de la peau, par application d'une composition cosmétique à base d'extrait aqueux de feuilles de pommier sur la zone de la peau nécessitant un tel traitement, ledit extrait comprenant des flavonoïdes, des polysaccharides et des sucres à raison de :
- flavonoïdes (exprimés en rutine) 0,5 -4,0 g/l
- polysaccharides (matières sèches) 2,0-8,0 %
- sucres totaux (matières sèches) 25,0 -40,0 %

## Patentansprüche

1. Verwendung eines Apfelbaumblattextraktes, umfassend Flavonoide, Polysaccharide und Zucker in der Menge von:
- Flavonoide (routinemäßig exprimiert) 0,5 - 4,0 g/l
- Polysaccharide (Trockenmassen) 2,0 - 8,0 %
- Gesamtzucker (Trockenmassen) 25,0 - 40,0 %
zur Herstellung einer kosmetischen Hautstraffungs-Zusammensetzung

2. Verwendung nach Anspruch 1, **dadurch gekennzeichnet, dass** der Extrakt auf der Basis von Apfelbaumblättern der Arten Pyrus malus oder Malus sylvestris Mill., Malus domestica, Malus pumila, Malus floribunda oder Malus coronaria erhalten wird.

3. Verfahren nach einem beliebigen der vorhergehenden Ansprüche, **dadurch gekennzeichnet, dass** der Extrakt die folgenden Eigenschaften aufweist:
pH 4,5 - 6,5
- Brechungsindex bei 22 ºC 1,330 - 1,380
- Dichte bei 20 ºC 0,950 - 1,050
- Resttrockenmassen 1,5 - 3,0 %

4. Verwendung nach einem beliebigen der vorhergehenden Ansprüche, **dadurch gekennzeichnet, dass** der Gehalt an Apfelbaumblattextrakten im Bereich zwischen 0,5 und 20 Gew% mit Bezug auf das Gesamtgewicht der Zusammensetzung liegt.

5. Verwendung nach Anspruch 4, **dadurch gekennzeichnet, dass** der Gehalt an Apfelbaumblattextrakt im Bereich zwischen 1 und 10 Gew% mit Bezug auf das Gesamtgewicht der Zusammensetzung liegt.

6. Verfahren zur nicht therapeutischen kosmetischen Hautstraffungsbehandlung durch Anwendung einer kosmetischen Zusammensetzung auf der Basis eines wässrigen Apfelbaumblattextrakte s auf den Bereich der Haut, der eine derartige Behandlung erfordert, wobei der Extrakt Flavonoide, Polysaccharide und Zucker umfasst in der Menge von:
- Flavonoide (routinemäßig exprimiert) 0,5 - 4,0 g/l
- Polysaccharide (Trockenmassen) 2,0 - 8,0 %
- Gesamtzucker (Trockenmassen) 25,0 - 40,0 %

## Claims

1. Use of an aqueous extract of apple tree leaves comprising flavonoids, polysaccharides and sugars in a proportion of:
- flavonoids (expressed in rutin) 0.5 - 4.0 g/l
- polysaccharides (dry matter) 2.0 - 8.0 %
- total sugars (dry matter) 25.0 - 40.0 %
to prepare a cosmetic skin firming composition.

2. The use according to claim 1, **characterized in that** said extract is obtained from leaves of apple trees of the species *Pyrus malus* or *Malus sylvestris Mill., Malus domestica, Malus pumila, Malus floribunda* or *Malus coronaria.*

3. The use according to any of the preceding claims, **characterized in that** said extract has the following characteristics:
- pH 4.5 - 6.5
- refractive index at 22°C 1.330 - 1.380
- density at 20 °C 0.950 - 1.050
- residual dry matter 1.5 - 3.0 %.

4. The use according to any of the preceding claims, **characterized in that** the content of extract of apple tree leaves is between 0.5 and 20 % by weight relative to the total weight of the composition.

5. The use (1) according to claim 4, **characterized in that** the content of extract of apple tree leaves is between 1 and 10 % by weight relative to the total weight of the composition.

6. Non-therapeutic cosmetic method for skin firming treatment, via application of a cosmetic composition containing an aqueous extract of apple tree leaves to a region of the skin in need of such treatment, said extract comprising flavonoids, polysaccharides and sugars in a proportion of:
- flavonoids 0.5 - 4.0 g/l
- polysaccharides (dry matter) 2.0 - 8.0 %
- total sugars (dry matter) 25.0 - 40.0 %
